# EUROPEAN PATENT APPLICATION

(11) **EP 3 885 363 A1**
(43) Date of publication of application: **29.09.2021**
(21) Application number: 19888178.1
(22) Date of filing: 06.11.2019
(51) Int. Cl.: C07K 16/26, C12N 5/20, G01N 33/74, G01N 33/577

(54) **SPECIFIC ANTIBODY FOR AMH, AND USES THEREOF**

(30) Priority: 20.11.2018 CN 201811382047
(71) Applicant: Xiamen Innodx Biotech Co., Ltd, Xiamen, Fujian 361022 (CN); Xiamen University, Xiamen, Fujian 361005 (CN)
(72) Inventor: XIONG, Junhui, Xiamen, Fujian 361022 (CN); CHEN, Zimin, Xiamen, Fujian 361022 (CN); XU, Weiling, Xiamen, Fujian 361022 (CN); WANG, Long, Xiamen, Fujian 361022 (CN); KE, Qishen, Xiamen, Fujian 361022 (CN); LI, Lihua, Xiamen, Fujian 361022 (CN); SONG, Liuwei, Xiamen, Fujian 361022 (CN); SUN, Xudong, Xiamen, Fujian 361022 (CN); GE, Shengxiang, Xiamen, Fujian 361005 (CN)
(74) Representative: De Vries & Metman
(86) International application number: PCT/CN2019/115998
(87) International publication number: WO 2020/103691

(57) **Abstract**

The present invention belongs to the field of biomedical laboratory science, and provides a specific antibody for AMH and uses thereof.

Sequence list

<110>  Xiamen Innodx Biotech Co., Ltd.
       University of Xiamen

<120>  Specific antibody against human anti-Mullerian hormone and
application thereof

<130>  IEC190137PCT

<160>  8

<170>  PatentIn version 3.5

<210>  1
<211>  8
<212>  PRT
<213>  Artificial Sequence

<400>  1

Gly Phe Ala Phe Ser Thr Tyr Asp
1               5


<210>  2
<211>  8
<212>  PRT
<213>  Artificial Sequence

<400>  2

Ile Ser Pro Gly Gly Gly Ala Thr
1               5


<210>  3
<211>  11
<212>  PRT
<213>  Artificial Sequence

<400>  3

Ala Gly Arg Arg Asp Tyr Tyr Gly Met Asp Tyr
1               5                   10


<210>  4
<211>  11
<212>  PRT
<213>  Artificial Sequence

<400>  4

Gln Ser Leu Val His Ser Asn Gly Asn Thr Tyr
1               5                   10


<210>  5
<211>  3
<212>  PRT
<213>  Artificial Sequence

<400>  5

Lys Val Ser
1



<210>  6
<211>  9
<212>  PRT
<213>  Artificial Sequence

<400>  6

Ser Gln Ser Thr His Val Pro Trp Thr
1               5



<210>  7
<211>  118
<212>  PRT
<213>  Artificial Sequence

<400>  7

Glu Val Gln Leu Val Glu Ser Gly Gly Gly Leu Val Lys Pro Gly Gly
1               5                   10                  15


Ser Leu Lys Leu Ser Cys Ala Ala Ser Gly Phe Ala Phe Ser Thr Tyr
            20                  25                  30


Asp Met Ser Trp Val Arg Gln Thr Pro Glu Lys Arg Leu Glu Trp Val
        35                  40                  45


Ala Tyr Ile Ser Pro Gly Gly Gly Ala Thr Tyr Tyr Pro Asp Thr Val
    50                  55                  60


Lys Gly Arg Phe Thr Ile Ser Arg Asp Asn Ala Lys Asn Thr Leu Tyr
65                  70                  75                  80


Leu Gln Met Thr Ser Leu Lys Ser Glu Asp Thr Ala Met Tyr Tyr Cys
                85                  90                  95


Ala Gly Arg Arg Asp Tyr Tyr Gly Met Asp Tyr Trp Gly Gln Gly Thr
            100                 105                 110


Ser Val Thr Val Ser Ser
        115



<210>  8
<211>  112
<212>  PRT
<213>  Artificial Sequence

<400>  8

Asp Val Val Met Thr Gln Thr Pro Leu Ser Leu Pro Val Ser Leu Gly
1               5                   10                  15

Asp Gln Ala Ser Ile Ser Cys Arg Ser Ser Gln Ser Leu Val His Ser
            20                  25                  30

Asn Gly Asn Thr Tyr Leu His Trp Tyr Leu Gln Lys Pro Gly Gln Ser
        35                  40                  45

Pro Lys Leu Leu Ile Tyr Lys Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Leu Gly Val Tyr Phe Cys Ser Gln Ser
                85                  90                  95

Thr His Val Pro Trp Thr Phe Gly Gly Gly Thr Lys Leu Glu Ile Lys
            100                 105                 110

## Description

### Technical Field

the present invention pertains to the field of biomedical testing, and mainly relates to a specific antibody against human anti-Mullerian hormone and application thereof.

### Background Art

Anti-Mullerian hormone (AMH) is a type of monoglycoprotein secreted by immature sertoli cells of testis and granular cells of ovarian growing follicles, belongs to transforming growth factor β superfamily, and has important functions of inhibiting the development of male Mullerian ducts and regulating the development of male and female germ cells and gonad. In recent years, scholars from various countries have conducted extensive research on its role in the field of female reproduction. In women, AMH can regulate follicular development, reflect ovarian reserve function, predict the responsiveness of ovaries to superovulation, and has certain values for the study of reproductive endocrine diseases. At present, there are many records about the detection of AMH by immunoassay. For example, US patent 7,897,350 describes a composition and method for detecting AMH in a sample, wherein an antibody binds to a mature region or C-terminal region of AMH. European patent EP2161579A describes a method for detecting or quantifying at least one biologically active form of AMH in a sample, such as cleaved AMH or C-terminal AMH, and antibodies bound thereto.

In addition, there are also some other publications that disclose antibodies against N-terminal region of AMH. For example, Hudson et al. (1990) J. Clin. Endocrin. Metab. 70: 16-22 describe an immunoassay technique in which two monoclonal antibodies are directed against precursor and N-terminal region of AMH, respectively. Long et al. (2000) J. CIin. Endocrin. Metab. 85:540-544 describes an immunoassay technique that also uses two monoclonal antibodies, one binds to N-terminal epitope of AMH and the other binds to C-terminal region of AMH. In addition, WO2014204327A1 also discloses an antibody against N-terminal region of AMH, wherein the N-terminal region refers to residues 1-451 of human propeptide or residues 25-451 when leader sequence is removed. However, when the aforementioned anti-AMH antibodies are used to detect antigens, there are problems of poor detection stability and low sensitivity.

Therefore, there is a need in the art to develop specific antibodies against human anti-Mullerian hormone.

### Contents of the present invention

The first aspect of the present invention provides a monoclonal antibody or antigen-binding fragment thereof against human anti-Mullerian hormone, the antibody or antigen-binding fragment thereof specifically binds to an epitope of human anti-Mullerian hormone, the epitope is selected from the group consisting of an epitope contained in a sequence spanning amino acid residues 26 to 85 of anti-Mullerian hormone, an epitope contained in a sequence spanning amino acid residues 38 to 45 of anti-Mullerian hormone, an epitope contained in a sequence spanning amino acid residues 40 to 46 of anti-Mullerian hormone, an epitope contained in a sequence spanning amino acid residues 37 to 44 of anti-Mullerian hormone, an epitope contained in a sequence spanning amino acid residues 39 to 45 of anti-Mullerian hormone, an epitope contained in a sequence spanning amino acid residues 36 to 47 of anti-Mullerian hormone, an epitope contained in a sequence spanning amino acid residues 37 to 46 of Mullerian hormone. Preferably, the antibody or antigen-binding fragment thereof specifically binds to an epitope contained in a sequence spanning amino acid residues 38 to 45 of anti-Mullerian hormone.

The monoclonal antibody of the present invention specifically recognizes an epitope of human anti-Mullerian hormone and does not cross-react with an anti-Mullerian hormone derived from other species, including goat anti-Mullerian hormone, owl anti-Mullerian hormone, bovine anti-Mullerian hormone, horse anti-mullerian hormone, monkey anti-mullerian hormone, canine anti-mullerian hormone, deer anti-mullerian hormone, mouse anti-mullerian hormone, guinea pig anti-mullerian hormone, etc. In certain preferred embodiments, the antibody of the present invention is a chimeric antibody or a humanized antibody. In certain preferred embodiments, the antigen-binding fragment of the present invention is selected from scFv, Fab, Fab', (Fab')₂, Fv fragment, diabody.

In a specific embodiment, the present invention provides a monoclonal antibody against human anti-Mullerian hormone, which specifically binds to an epitope contained in a sequence spanning amino acid residues 38 to 45 of anti-Mullerian hormone.

Preferably, the antibody comprises heavy chain CDR sequences CDR1 GFAFSTYD, CDR2 ISPGGGAT, CDR3 AGRRDYYGMDY as shown in SEQ ID NO: 1 to 3, respectively; and light chain CDR sequences CDR1 QSLVHSNGNTY, CDR2 KVS, CDR3 SQSTHVPWT as shown in SEQ ID NO: 4 to 6, respectively.

More preferably, the antibody comprises a heavy chain variable region sequence as shown in SEQ ID NO: 7
EVQLVESGGGLVKPGGSLKLSCAASGFAFSTYDMSWVRQTPEKRLEWVAYISPGGG ATYYPDTVKGRFTISRDNAKNTLYLQMTSLKSEDTAMYYCAGRRDYYGMDYWGQGTS VTVSS, and a light chain variable region sequence as shown in SEQ ID NO: 8

DVVMTQTPLSLPVSLGDQASISCRSSQSLVHSNGNTYLHWYLQKPGQSPKLLIYKVS
NRFSGVPDRFSGSGSGTDFTLKISRVEAEDLGVYFCSQSTHVPWTFGGGTKLEIK.

In a preferred embodiment, the monoclonal antibody of the present invention is a monoclonal antibody secreted by a hybridoma cell line C9F2-2 deposited in the China Center for Type Culture Collection (Wuhan University, Wuhan, China) on August 19, 2018, and the hybridoma cell line has a deposit number of CCTCC NO: C2018183.

In another aspect, the present invention provides an isolated nucleic acid molecule comprising a nucleotide sequence encoding the antibody or antigen-binding fragment thereof or heavy chain variable region and/or light chain variable region thereof according to the present invention. In certain preferred embodiments, the isolated nucleic acid molecule encodes the antibody or antigen-binding fragment thereof or heavy chain variable region and/or light chain variable region thereof according to the present invention.

In another aspect, the present invention provides a vector (e.g., a cloning vector or expression vector), which comprises the isolated nucleic acid molecule of the present invention. In certain preferred embodiments, the vector of the present invention is, for example, a plasmid, cosmid, phage and the like. In certain preferred embodiments, the vector is capable of expressing the antibody or antigen-binding fragment thereof according to the present invention in a subject (e.g., a mammal, such as a human).

In another aspect, the present invention provides a host cell comprising the isolated nucleic acid molecule of the present invention or the vector of the present invention. Such host cell includes, but is not limited to, prokaryotic cell such as E. coli cell, and eukaryotic cell such as yeast cell, insect cell, plant cell and animal cell (e.g., mammalian cell, such as mouse cell, human cell, etc.). In certain preferred embodiments, the host cell of the present invention is a mammalian cell.

The antibody or antigen-binding fragment thereof according to the present invention can specifically bind to human anti-Mullerian hormone, and can be used to detect the presence or level of human anti-Mullerian hormone in a sample.

Therefore, in another aspect, the present invention provides a kit comprising the antibody or antigen-binding fragment thereof according to the present invention. In certain preferred embodiments, the antibody or antigen-binding fragment thereof according to the present invention bears a detectable label. In the present invention, the detectable label may be any substance that can be detected by fluorescence, spectroscopy, photochemistry, biochemistry, immunology, electrical, optical or chemical means. It is particularly preferable that such a label can be applied to immunological detection (e.g., enzyme-linked immunoassay, radioimmunoassay, fluorescence immunoassay, chemiluminescence immunoassay, etc.). Such label is well known in the art and includes, but is not limited to, enzyme (e.g., horseradish peroxidase, alkaline phosphatase, β-galactosidase, urease, glucose oxidase, etc.), radionuclide (e.g., ³H, ¹²⁵I, ³⁵S, ¹⁴C or ³²P), fluorescent dye (e.g., fluorescein isothiocyanate (FITC), fluorescein, tetramethylrhodamine isothiocyanate (TRITC), phycoerythrin (PE), Texas red, rhodamine, quantum dot or cyanine dye derivative (e.g., Cy7, Alexa 750), luminescent substance (e.g., chemiluminescent substance, such as acridinium ester compound), magnetic bead (e.g., Dynabeads®), calorimetric label such as colloidal gold or colored glass or plastic (e.g., polystyrene, polypropylene, latex, etc.) bead, and biotin used to bind an avidin (e.g., streptavidin) modified by the above label. Patents teaching the use of such label include, but are not limited to, US patents 3,817,837; 3,850,752; 3,939,350; 3,996,345; 4,277,437; 4,275,149; and 4,366,241 (all incorporated herein by reference). The label covered in the present invention can be detected by methods known in the art. For example, radioactive label can be detected using photographic film or a scintillation calculator, and fluorescent label can be detected using a light detector to detect the emitted light. Enzyme label is generally detected by providing a substrate to the enzyme and detecting the reaction product produced by the action of the enzyme on the substrate, and calorimetric label is detected by simply visualizing colored label. In certain embodiments, the detectable labels as described above can be attached to the antibody or antigen-binding fragment thereof according to the present invention through linkers of different lengths to reduce potential steric hindrance.

In another aspect, the present invention provides a method for detecting the presence or level of human anti-Mullerian hormone in a sample, which comprises a step of using the antibody or antigen-binding fragment thereof according to the present invention. In a preferred embodiment, the antibody or antigen-binding fragment thereof according to the present invention also bears a detectable label. In another preferred embodiment, the method further comprises using a reagent bearing a detectable label to detect the antibody or antigen-binding fragment thereof according to the present invention. The method can be used for diagnostic purposes, or for non-diagnostic purposes (e.g., the sample is a cell sample, not a sample from a patient).

In another aspect, there is provided a use of the antibody or antigen-binding fragment thereof according to the present invention in manufacture of a kit for detecting the presence or level of human anti-Mullerian hormone in a sample.

In a specific embodiment, the present invention provides a method for detecting AMH and fragment thereof in a biological sample derived from a human body fluid, the method comprising:
allowing the sample to contact with at least two antibodies or antigen-binding fragments thereof against different epitopes of anti-Mullerian hormone, and qualitatively or quantitatively detecting the binding of the at least two antibodies to the anti-Mullerian hormone or the fragments, wherein the binding indicates the presence or concentration of anti-Mullerian hormone or the fragment in the sample;
wherein at least one of the antibodies or antigen-binding fragments thereof is directed against an epitope contained in a sequence of amino acid residues 37 to 46 of anti-Mullerian hormone.

In a preferred embodiment, one of the antibodies or antigen-binding fragments thereof is directed against an epitope contained in a sequence of amino acid residues 38 to 45 of anti-Mullerian hormone, and the other antibody or antigen-binding fragment thereof is directed against an epitope contained in a sequence of amino acid residues 358 to 369 of anti-Mullerian hormone, or an epitope contained in a sequence of amino acid residues 491 to 502 of anti-Mullerian hormone, or an epitope contained in a sequence of amino acid residues 260 to 271 of anti-Mullerian hormone, or an epitope contained in a sequence of amino acid residues 330 to 343 of anti-Mullerian hormone.

In a preferred embodiment of the method of the present invention, at least one of the at least two antibodies or antigen-binding fragments thereof is immobilized on a solid surface. Preferably, the other or at least one of the other antibodies is labeled, preferably labeled with a chemiluminescent or fluorescent dye through a covalent bond.

In a specific embodiment of the method of the present invention, an antibody or antigen-binding fragment thereof against an epitope contained in a sequence spanning amino acid residues 38 to 45 of anti-Mullerian hormone is immobilized on a solid surface .

### Definition of Terms

In the present invention, unless otherwise specified, the scientific and technical terms used herein have the meanings commonly understood by those skilled in the art. Moreover, the operating steps of cell culture, biochemistry, nucleic acid chemistry, immunology laboratory and the like used in the context are all routine steps widely used in the corresponding fields. At the same time, in order to better understand the present invention, definitions and explanations of related terms are provided below.

As used herein, the term "antibody" refers to an immunoglobulin molecule usually composed of two pairs of polypeptide chains (each pair has a light chain (LC) and a heavy chain (HC)). Antibody light chains can be classified into κ (kappa) and λ (lambda) light chains. Heavy chains can be classified as µ, δ, γ, α, or ε, and the isotype of antibody is defined as IgM, IgD, IgG, IgA and IgE, respectively. Within the light and heavy chains, the variable and constant regions are connected by a "J" region of about 12 or more amino acids, and the heavy chain also comprises a "D" region of about 3 or more amino acids. Each heavy chain is composed of a heavy chain variable region (VH) and a heavy chain constant region (CH). The heavy chain constant region is composed of 3 domains (CH1, CH2, and CH3). Each light chain is composed of a light chain variable region (VL) and a light chain constant region (CL). The light chain constant region is composed of a domain CL. Constant domains are not directly involved in the binding of antibodies and antigens, but exhibit a plurality of effector functions, such as mediating the binding of immunoglobulins and host tissues or factors, including various cells of immune system (e.g., effector cells) and first component (C1q) of classical complement system. VH and VL regions can also be subdivided into regions with hyperdenaturation (which are called complementarity determining regions (CDR)), interspersed with more conservative regions that are called framework regions (FR). Each V_{H} and V_{L} is composed of 3 CDRs and 4 FRs that are arranged from amino terminal to carboxy terminal in the following sequence: FR1, CDR1, FR2, CDR2, FR3, CDR3, FR4. The variable regions (VH and VL) of each heavy chain/light chain pair respectively form an antigen binding site. The assignment of amino acids in each region or domain can follow the definitions of Kabat, Sequences of Proteins of Immunological Interest (National Institutes of Health, Bethesda, Md. (1987 and 1991)), or Chothia & Lesk (1987) J. Mol. Biol. 196 :901-917; Chothia et al. (1989) Nature 342:878-883.

As used herein, the term "complementarity determining region" or "CDR" refers to amino acid residues in variable region of antibody that are responsible for antigen binding. The precise boundaries of these amino acid residues can be defined by various well-known numbering systems, for example, according to the definitions of Kabat numbering system (Kabat et al., Sequences of Proteins of Immunological Interest, 5th Ed. Public Health Service, National Institutes of Health, Bethesda, Md., 1991), Chothia numbering System (Chothia & Lesk (1987) J. Mol. Biol. 196:901-917; Chothia et al. (1989) Nature 342:878-883) or IMGT numbering system (Lefranc et al., Dev. Comparat. Immunol. 27 :55-77, 2003). For a given antibody, those skilled in the art will easily identify the CDRs defined by each numbering system. Moreover, the correspondence between different numbering systems is well known to those skilled in the art (e.g., see: Lefranc et al., Dev. Comparat. Immunol. 27:55-77, 2003).

In the present invention, the CDRs contained in the antibody or antigen-binding fragment thereof of the present invention can be determined according to various numbering systems known in the art. In certain embodiments, the CDRs contained in the antibody or antigen-binding fragment thereof of the present invention are preferably determined by the Kabat, Chothia, or IMGT numbering system. In certain embodiments, the CDRs contained in the antibody or antigen-binding fragment thereof of the present invention are preferably determined by the Kabat numbering system.

As used herein, the term "framework region" or "FR" residues refers to those amino acid residues in variable region of antibody other than the CDR residues as defined above.

The term "antibody" is not limited by any specific method of producing antibodies. For example, it comprises recombinant antibody, monoclonal antibody, and polyclonal antibody. The antibody may be an antibody of different isotypes, for example, IgG (e.g., IgG1, IgG2, IgG3 or IgG4 subtype), IgA1, IgA2, IgD, IgE or IgM antibody.

As used herein, the term "antigen-binding fragment" of an antibody refers to a polypeptide comprising a fragment of a full-length antibody, which retains the ability to specifically bind to the same antigen to which the full-length antibody binds, and/or competes with the full-length antibody for specific binding to antigen, and which is also called "antigen binding component". See generally, Fundamental Immunology, Ch. 7 (Paul, W., ed., 2nd edition, Raven Press, NY (1989), which is incorporated herein by reference in its entirety for all purposes. The antigen-binding fragments of antibody can be produced by recombinant DNA technology or by enzymatic or chemical cleavage of intact antibody. Non-limiting examples of antigen-binding fragments include Fab, Fab', F(ab')2, Fd, Fv, dAb and complementarity determining region (CDR) fragments, single-chain antibody (e.g., scFv), chimeric antibody, diabody, linear antibody, nanobody (which technology is from Domantis), domain antibody (which technology is from Ablynx), probody and such polypeptides, which contains at least a portion of antibody sufficient to confer specific antigen-binding ability to polypeptide. Engineered antibody variants are reviewed by Holliger et al., 2005; Nat Biotechnol, 23: 1126-1136.

Conventional techniques known to those skilled in the art (e.g., recombinant DNA technology or enzymatic or chemical fragmentation) can be used to obtain antigen-binding fragments (e.g., the above-mentioned antibody fragments) from a given antibody (e.g., the antibody provided by the present invention), and to specifically screen for antigen-binding fragments of antibody in the same manner as used for intact antibody. Herein, unless the context clearly dictates otherwise, when the term "antibody" is referred to, it comprises not only intact antibody but also antigen-binding fragments of antibody.

As used herein, the terms "monoclonal antibody", "McAb" and "mAb" have the same meaning and are used interchangeably, which refer to one antibody or one fragment of antibody among a group of highly homologous antibody molecules, that is, a group of identical antibody molecules except for natural mutations that may occur spontaneously. The monoclonal antibody has high specificity for a single epitope on antigen. Polyclonal antibody is relative to monoclonal antibody, which usually comprises at least two or more different antibodies, and these different antibodies usually recognize different epitopes on antigen. In addition, the modifier "monoclonal" only indicates that the antibody is characterized as being obtained from a group of highly homologous antibodies, and cannot be understood as requiring any specific method to prepare the antibody.

The monoclonal antibody of the present invention can be prepared by a variety of techniques, for example, hybridoma technology (see, for example, Kohler et al. Nature, 256:495, 1975), recombinant DNA technology (see, for example, US Patent Application 4,816,567), or phage antibody library technology (see, for example, Clackson et al. Nature352:624-628, 1991, or Marks et al. J. Mol. Biol. 222:581-597, 1991).

The antibody can be purified by known techniques, for example, affinity chromatography using protein A or protein G. Subsequently or as an alternative, a specific antigen (a target molecule recognized by the antibody) or an epitope thereof can be immobilized on column, and the immunospecific antibody can be purified by immunoaffinity chromatography. Reference for the purification of immunoglobulin may be seen in, for example, D. Wilkinson (The Scientist, published by The Scientist, Inc., Philadelphia Pa., Vol. 14, No. 8 (Apr. 17, 2000), pp. 25-28).

As used herein, the term "specific binding" refers to a non-random binding reaction between two molecules, such as the reaction between an antibody and an antigen to which it targets. The strength or affinity of a specific binding interaction may be expressed by an equilibrium dissociation constant (K_{D}) of the interaction. In the present invention, the term "K_{D}" refers to a dissociation equilibrium constant of a specific antibody-antigen interaction, which is used to describe the binding affinity between the antibody and the antigen. The smaller the equilibrium dissociation constant, the tighter the antibody-antigen binding, and the higher the affinity between the antibody and the antigen. In some embodiments, an antibody that specifically binds to a certain antigen (or an antibody that is specific to a certain antigen) means that the antibody has an affinity(K_{D}) of less than about 10⁻⁹ M, for example, less than about 10⁻⁹ M, 10⁻¹⁰ M, 10⁻¹¹ M or 10⁻¹² M or less when it binds the antigen. The specific binding properties between two molecules can be measured by methods known in the art, for example, measured by surface plasmon resonance (SPR) in a BIACORE instrument.

The embodiments of the present invention will be described in detail below in conjunction with examples. However, those skilled in the art will understand that the following examples are only used to illustrate the present invention, but not to limit the scope of the present invention. According to the following detailed description of preferred embodiments, the various objects and advantageous aspects of the present invention will become implementable for those skilled in the art.

### Example 1. Production of specific antibody against human anti-Mullerian hormone

1. 6 female BALB/c mice were selected and subcutaneously immunized with AMH antigen emulsified by complete Freund's adjuvant (CFA) (Sigma, St. Louis, MO), and then immunized 2 weeks later with the same antigen emulsified by incomplete Freund's adjuvant ( IFA) (Sigma, St. Louis, MO), and the immunization was continued for 2 injections at 2 weeks intervals.
2. Blood samples were obtained from capillaries behind eyes at 6 and 8 weeks after the initial immunization and at the time of execution, the execution was carried out at 9 weeks later, and spleen was taken out at the same time. The spleen cells were fused with SP2/0 myeloma cells, cultured in a CO₂ incubator for 7 days, and the cell supernatant was changed. After 2 days, the generated hybridoma cell lines were tested.
3. Indirect screening method was used, in which AMH antigen was diluted with carbonic acid buffer (20 mmol/L CB pH 9.6) and then coated on a polyvinyl chloride plate, then a sample to be tested was added, and finally HRP-labeled goat anti-mouse secondary antibody (stored in 20 mmol/L PBS pH 7.4) was added, and the cell lines were screened according to their abilities of secreting antibodies that bound to the immobilized recombinant human AMH (anti-Mullerian Hormone).
4. After the cells were subjected to stable cloning for 3 times, a stable cell line was obtained, which was cultured in large numbers, and some cells were cryopreserved for breed conservation. Mice were induced to produce ascites, the ascites was subjected to affinity column purification to obtain 5 monoclonal antibodies, C8H3-2, C9F2-2, 7D11-1, C17B10, G8B11, with a purity of greater than 90%, which were stored in 20 mmol PBS solution. The monoclonal antibody C9F2-2 was deposited in the China Center for Type Culture Collection on August 19, 2018, and the access number of the hybridoma cell line C9F2-2 was CCTCC NO: C2018183.

### Example 2:

Evaluation of monoclonal antibody and identification of truncated antigen:
1. AMH antigen was diluted with carbonic acid buffer (20 mmol/L CB, pH 9.6) and then coated on a polyvinyl chloride plate. In the pretreatment, the antibody purified by the protein A column and the synthesized AMH truncated antigen sequences (see the last supplement for each synthetic sequence) were subjected to proportion incubation (100ng/ml antibody: 50ug/ml truncated antigen) for 30 minutes, that were test samples; the control sample was 100ng/ml antibody: PBS, incubation for 30 minutes, and then the test samples after incubation and the control sample were added to AMH antigen plate and incubated for 30 minutes, the plate was washed for 5 times, added with GAM-HRP (1/5000 dilution) and incubated for 30 minutes, the plate was washed for 5 times, and the substrate was added and incubated for 15 minutes. The values under wavelength of 450/620 were read by microplate reader, and the detected OD values were finally calculated and analyzed. The calculation formula was: (control sample OD value - test sample OD value)/control sample OD value, and the criterion for determination was as follows: more than 90%, which indicated a good competitive effect, showing that the antibody should recognize the epitope. The data were shown in Table 1 below.

| | C8H3-2 | C9F2-2 | 7D11-1 | C17B10 | G8B11 |
|---|---|---|---|---|---|
| AMH (1-560) | 90% | 92% | 97% | 94% | 96% |
| 26-85 | 99% | 99% | 99% | 93% | 99% |
| 86-185 | 6% | 2% | 12% | 4% | 1% |
| 171-270 | 3% | 0% | 7% | 4% | 3% |
| 256-355 | 3% | 1% | 7% | 4% | 8% |
| 341-440 | 7% | 10% | 11% | 3% | 6% |
| 426-525 | 3% | 1% | 11% | 7% | 8% |
| 461-560 | 7% | 6% | 11% | 7% | 10% |

The sequence of each truncated antigen is as follows:
26-85aa:
   RAEEPAVGTSGLIFREDLDWPPGSPQEPLCLVALGGDSNGSSSPLRVVGALSAYEQAFLG
86-185aa 
AVQRARWGPRDLATFGVCNTGDRQAALPSLRRLGAWLRDPGGQRLVVLHLEEVT
WEPTPSLRFQEPPPGGAGPPELALLVLYPGPGPEVTVTRAGLPGAQ
171-270aa 
GPEVTVTRAGLPGAQSLCPSRDTRYLVLAVDRPAGAWRGSGLALTLQPRGEDSRLS
TARLQALLFGDDHRCFTRMTPALLLLPRSEPAPLPAHGQLDTVP
256-355aa 
EPAPLPAHGQLDTVPFPPPRPSAELEESPPSADPFLETLTRLVRALRVPPARASAPRLA
LDPDALAGFPQGLVNLSDPAALERLLDGEEPLLLLLRPTAA
341-440aa 
DGEEPLLLLLRPTAATTGDPAPLHDPTSAPWATALARRVAAELQAAAAELRSLPGLP
PATAPLLARLLALCPGGPGGLGDPLRALLLLKALQGLRVEWRG
426-525aa 
LLLKALQGLRVEWRGRDPRGPGRAQRSAGATAADGPCALRELSVDLRAERSVLIPE
TYQANNCQGVCGWPQSDRNPRYGNHVVLLLKMQARGAALARPPC
461-560aa

PCALRELSVDLRAERSVLIPETYQANNCQGVCGWPQSDRNPRYGNHVVLLLKMQAR
GAALARPPCCVPTAYAGKLLISLSEERISAHHVPNMVATECGCR

2. After identification, there were 5 monoclonal antibodies that showed a strong reactivity in term of full-length AMH and a strong reactivity in term of 26-85 truncated antigen.

### Example 3:

Identification of monoclonal antibody epitope:
1. AMH antigen was diluted with carbonic acid buffer (20 mmol/L CB, pH 9.6) and then coated on a polyvinyl chloride plate. In the pretreatment, the antibody purified by the protein A column and the synthesized AMH truncated antigen sequences (see Table 3 for each synthetic sequence) were subjected to proportion incubation (100ng/ml antibody: 50ug/ml truncated antigen) for 30 minutes, that were test samples; the control sample was 100ng/ml antibody: PBS, incubation for 30 minutes, and then the test samples after incubation and the control sample were added to AMH antigen plate and incubated for 30 minutes, the plate was washed for 5 times, added with GAM-HRP (1/5000 dilution) and incubated for 30 minutes, the plate was washed for 5 times, and the substrate was added and incubated for 15 minutes. The values under wavelength of 450/620 were read by microplate reader, and the detected OD values were finally calculated and analyzed. The calculation formula was: (control sample OD value - test sample OD value)/control sample OD value, and the criterion for determination was as follows: more than 90%, which indicated a good competitive effect, showing that the antibody should recognize the epitope. The data were shown in Table 2 below.

| | C8H3-2 | C9F2-2 | 7D11-1 | C17B10 | G8B11 |
|---|---|---|---|---|---|
| 21-35 | 23% | 10% | 20% | 2% | 29% |
| 31-45 | 79% | 90% | 100% | 98% | 97% |
| 41-55 | 100% | 68% | 49% | 10% | 100% |
| 51-65 | -23% | 9% | 6% | -9% | 2% |
| 61-75 | 17% | 35% | 50% | 4% | 35% |
| 71-85 | 27% | 21% | 21% | 4% | 36% |
| 31-41 | 19% | 10% | 87% | -1% | 32% |
| 31-42 | 18% | 16% | 81% | 30% | 33% |
| 31-43 | 21% | 25% | 84% | 22% | 31% |
| 31-44 | 13% | 38% | 99% | 18% | 32% |
| 32-41 | 2% | 9% | 78% | -2% | 19% |
| 33-41 | 26% | 9% | 70% | 9% | 37% |
| 34-41 | -1% | 3% | 66% | 18% | 23% |
| 34-45 | 90% | 98% | 100% | 98% | 99% |
| 35-45 | 94% | 97% | 100% | 98% | 99% |
| 36-44 | 34% | 32% | 100% | 27% | 41% |
| 36-45 | 91% | 94% | 100% | 98% | 99% |
| 36-47 | 99% | 100% | 100% | 98% | 99% |
| 37-43 | 9% | 57% | 83% | 37% | 18% |
| 37-44 | 3% | 24% | 100% | 13% | 28% |
| 37-45 | 93% | 91% | 100% | 98% | 100% |
| 38-44 | 9% | 33% | 61% | 15% | 32% |
| 38-45 | 87% | 92% | 78% | 99% | 98% |
| 39-45 | 84% | 68% | 22% | 101% | 97% |
| 40-45 | 38% | -14% | 2% | 4% | 81% |
| 40-46 | 90% | -2% | 18% | 39% | 96% |
| 40-47 | 97% | -19% | 4% | 40% | 100% |
| 41-45 | 53% | 15% | 21% | 30% | 43% |
| 41-46 | 82% | 15% | 29% | 29% | 56% |
| 41-47 | 93% | 64% | 29% | 20% | 97% |
| 41-48 | 98% | -1% | 14% | 12% | 100% |
| 41-49 | 101% | 3% | 23% | 9% | 99% |
| 41-50 | 99% | 65% | 62% | 18% | 99% |
| 42-46 | 28% | -10% | 13% | -9% | 13% |
| 42-47 | 86% | 6% | 19% | -4% | 80% |
| 42-48 | 85% | 2% | 18% | 9% | 82% |
| 42-49 | 90% | -8% | 7% | -20% | 87% |
| 42-50 | 91% | 57% | 16% | -2% | 86% |
| 43-50 | 8% | 17% | 52% | 3% | 31% |
| 41-51 | 99% | 71% | 40% | 4% | 99% |
| 42-51 | 93% | 60% | 30% | 6% | 83% |
| 43-51 | -9% | -10% | 3% | -4% | 4% |

**Table 3**

| Name | Amino acid sequence |
|---|---|
| 21-35 | GTEALRAEEPAVGTS |
| 31-45 | AVGTSGLIFREDLDW |
| 41-55 | EDLDWPPGSPQEPLC |
| 51-65 | QEPLCLVALGGDSNG |
| 61-75 | GDSNGSSSPLRVVGA |
| 71-85 | RVVGALSAYEQAFLG |
| 31-41 | AVGTSGLIFRE |
| 31-42 | AVGTSGLIFRED |
| 31-43 | AVGTSGLIFREDL |
| 31-44 | AVGTSGLIFREDLD |
| 32-41 | VGTSGLIFRE |
| 33-41 | GTSGLIFRE |
| 34-41 | TSGLIFRE |
| 34-45 | TSGLIFREDLDW |
| 35-45 | SGLIFREDLDW |
| 36-44 | GLIFR EDLD |
| 36-45 | GLIFREDLDW |
| 36-47 | GLIFR EDLDW PP |
| 37-43 | LIFR EDL |
| 37-44 | LIFR EDLD |
| 37-45 | LIFREDLDW |
| 38-44 | IFR EDLD |
| 38-45 | IFREDLDW |
| 39-45 | FREDLDW |
| 40-45 | REDLDW |
| 40-46 | REDLDWP |
| 40-47 | REDLDWPP |
| 41-45 | EDLDW |
| 41-46 | EDLDWP |
| 41-47 | EDLDWPP |
| 41-48 | EDLDWPPG |
| 41-49 | EDLDWPPGS |
| 41-50 | EDLDWPPGSP |
| 42-46 | DLDWP |
| 42-47 | DLDWPP |
| 42-48 | DLDWPPG |
| 42-49 | DLDWPPGS |
| 42-50 | DLDWPPGSP |
| 43-50 | LDWPPGSP |
| 41-51 | EDLDWPPGSPQ |
| 42-51 | DLDWPPGSPQ |
| 43-51 | LDWPPGSPQ |

| | |
|---|---|
| 1. Through the analysis of experimental data, the monoclonal antibody epitopes were initially verified in C8H3-2(41-55), C9F2-2(31-45), 7D11-1(31-45), G8B11(31-45/41-55), C17B10(31-45). 2. Through the analysis of experimental data, the monoclonal antibody epitopes were verified in C8H3-2(40-46), C9F2-2(38-45), 7D11-1(37-44), G8B11(40-46/41-48/39-45), C17B10(39-45). | |

### Example 4

1. AMH antigen was diluted with carbonic acid buffer (20 mmol/L CB, pH 9.6) and then coated on a polyvinyl chloride plate. In the pretreatment, the antibody purified by the protein A column and the synthesized short peptides with different human AMH36-47 amino acid mutations were subjected to proportion incubation (100ng/ml antibody: 50ug/ml truncated antigen) for 30 minutes, that were test samples; the control sample was (100ng/ml antibody: PBS) and incubated for 30 minutes, and then the test samples after incubation and the control sample were added to AMH antigen plate and incubated for 30 minutes, the plate was washed for 5 times, added with GAM-HRP (1/5000 dilution) and incubated for 30 minutes, the plate was washed for 5 times, and the substrate was added and incubated for 15 minutes. The values under wavelength of 450/620 were read by microplate reader, and the detected OD values were finally calculated and analyzed. The calculation formula was: (control sample OD value - test sample OD value)/control sample OD value, and the criterion for determination was as follows: a value lower than 90% indicated the corresponding amino acid was a key amino acid, and the mutation has a great impact on the reaction of antibody. The data were shown in Table 4 below.

| Mutation site | Sequence after mutation | C8H3-2 | C9F2-2 | 7D11-1 | C17B10 | G8B11 |
|---|---|---|---|---|---|---|
| 36 | ALIFREDLDWPP | 100% | 100% | 101% | 99% | 99% |
| 37 | GAIFREDLDWPP | 99% | 97% | 100% | 99% | 99% |
| 38 | GLAFREDLDWPP | 100% | 96% | 84% | 100% | 100% |
| 39 | GLIAREDLDWPP | 98% | 9% | 100% | 91% | 88% |
| 40 | GLIFAEDLDWPP | 99% | 88% | 100% | 100% | 99% |
| 41 | GLIFRADLDWPP | 98% | 7% | 99% | 90% | 99% |
| 42 | GLIFREALDWPP | 18% | 98% | 69% | 48% | 96% |
| 43 | GLIFREDADWPP | 99% | 94% | 100% | 100% | 100% |
| 44 | GLIFREDLAWPP | 99% | 64% | 99% | 97% | 99% |
| 45 | GLIFREDLDAPP | 27% | 100% | 12% | 24% | 29% |
| 46 | GLIFREDLDWAP | 99% | 99% | 98% | 84% | 99% |
| 47 | GLIFREDLDWPA | 99% | 99% | 99% | 99% | 99% |

2. From the results of the analysis, it could be seen:
the key impact sites of C8H3-2 were sites 42, 45;
the key impact sites of C9F2-2 were sites 39, 41, 44;
the key impact sites of 7D11-1 were sites 38, 42, 45;
the key impact sites of C17B10 were sites 42, 45, 46;
the key impact sites of G8B11 were were sites 39, 45;
which were consistent with the above epitope identification results.

### Example 5

Evaluation of monoclonal antibody species specificity:
1. AMH antigen was diluted with carbonic acid buffer (20 mmol/L CB, pH 9.6) and then coated on a polyvinyl chloride plate. In the pretreatment, the antibody purified by the protein A column and the synthesized short peptides with different human AMH36-47 amino acid mutations (see Table 6 for each synthesized sequence) were subjected to proportion incubation (100ng/ml antibody: 50ug/ml truncated antigen) for 30 minutes, that were test samples; the control sample was (100ng/ml antibody: PBS) and incubated for 30 minutes, and then the test samples after incubation and the control sample were added to AMH antigen plate and incubated for 30 minutes, the plate was washed for 5 times, added with GAM-HRP (1/5000 dilution) and incubated for 30 minutes, the plate was washed for 5 times, and the substrate was added and incubated for 15 minutes. The values under wavelength of 450/620 were read by microplate reader, and the detected OD values were finally calculated and analyzed. The calculation formula was: (control sample OD value - test sample OD value)/control sample OD value, and the criterion for determination was as follows: 90% or more, which represented that there was cross-reaction. The data were shown in Table 5 below.

| Sequence of different species | C8H3 | C9F2-2 | 7D11 | 17B10 | G8B11 |
|---|---|---|---|---|---|
| AMH3647-1 | 1% | -6% | 46% | 41% | 46% |
| AMH3647-2 | 77% | 11% | 4% | 85% | 99% |
| AMH3647-3 | 7% | 8% | -3% | -4% | 24% |
| AMH3647-4 | 13% | 22% | 99% | 93% | 86% |
| AMH3647-5 | 83% | 32% | 98% | 98% | 99% |
| AMH3647-6 | 12% | 3% | -17% | -8% | 10% |
| AMH3647-7 | 19% | 6% | -4% | 19% | 19% |
| AMH3647-8 | 13% | 8% | 62% | 33% | 48% |
| AMH3647-9 | 15% | -3% | 58% | 30% | 38% |
| AMH3647-10 | 19% | 8% | -6% | -3% | 31% |
| AMH3647-11 | 24% | 5% | 30% | 13% | 29% |
| AMH3647-12 | 41% | 5% | 29% | 16% | 48% |
| AMH3647-13 | 27% | 11% | 31% | 26% | 35% |
| AMH3647-14 | 28% | 29% | 42% | 29% | 86% |
| AMH3647-15 | 65% | 2% | -16% | 10% | 78% |
| AMH3647-16 | 30% | -2% | -18% | 2% | 100% |
| AMH3647-17 | 92% | 9% | -23% | 37% | 99% |
| AMH3647-18 | 42% | 19% | 56% | 64% | 95% |
| AMH3647-19 | 35% | -1% | -16% | 44% | 93% |
| AMH3647-20 | 21% | 3% | 59% | 61% | 32% |
| AMH3647-21 | 15% | 10% | 18% | -7% | 16% |
| AMH3647-22 | 75% | -9% | 60% | 20% | 82% |
| AMH3647-23 | 28% | -8% | 46% | 1% | 0% |
| AMH3647-24 | 94% | 19% | 55% | 99% | 99% |
| AMH3647-25 | 37% | -2% | 78% | 34% | 95% |
| AMH3647-26 | 93% | 61% | 42% | 99% | 99% |
| AMH3647-27 | 100% | 100% | 99% | 99% | 99% |

**Table 6**

| Sequence | Species | Synthesized sequence No. |
|---|---|---|
| ALIFQQDWDWPL | Deer | AMH3647-1 |
| DLIFHEDWAWPP | Beaver | AMH3647-2 |
| GLIFFEDGIWPL | Hamster | AMH3647-3 |
| GLIFHEDWDWLP | Lemur | AMH3647-4 |
| GLIFHEDWDWPP | Diademed Sifaka | AMH3647-5 |
| GLIFHKDWDWQP | Otolemur | AMH3647-6 |
| GLIFHPDWDWQP | Cat | AMH3647-7 |
| GLIFHQDWDWPA | Myotis | AMH3647-8 |
| GLIFHQDWDWSP | Horseshoe bat | AMH3647-9 |
| GLIFHWDWNWPP | Pig | AMH3647-10 |
| GLIFLEDGIWPP | Neotoma | AMH3647-11 |
| GLIFLEDGLWPP | Gerbil | AMH3647-12 |
| GLIFLEDGVWPP | Rat | AMH3647-13 |
| GLIFLQDGMWSP | Little mole rat | AMH3647-14 |
| GLIFPEDQVWPP | Guinea pig | AMH3647-15 |
| GLIFPEDWVWPP | Degu | AMH3647-16 |
| GLIFPEDLDWPP | Chimpanzee | AMH3647-17 |
| GLIFRQDWDWPP | Simum | AMH3647-18 |
| GLLFQPDWDWPP | Dog | AMH3647-19 |
| TLIFQQDWDWPL | Goat | AMH3647-20 |
| TLIFQQGWDWPL | Sheep | AMH3647-21 |
| GLIFLEDELWPP | Mouse | AMH3647-22 |
| ALIFQQAWDWPL | Cattle | AMH3647-23 |
| GLIFGEDVDWPP | Owl | AMH3647-24 |
| GLIFHQDWDWPP | Horse | AMH3647-25 |
| GLIFREDLGWPP | Monkey | AMH3647-26 |
| GLIFREDLDWPP | Human | AMH3647-27 |

2. Through the analysis of experimental data, our C9F2-2 epitope 38-45 monoclonal antibody had no cross-reaction with camel, owl, cattle, monkey, horse, mouse, dog and sheep sequences in term of species-specificity.

### Example 7

Evaluation of monoclonal antibody paired enzyme immunity:
1. Phosphate buffer (20 mmol/LPB, pH7.4) for monocloning antibody was diluted and coated on a polyvinyl chloride plate, and a monoclonal antibody was labeled with horseradish peroxidase (labeled monoclonal antibody was F10G7-2, Xiamen Wantaikairui Biotechnology Co., Ltd., catalog number M3382; which specifically bound to amino acids 260 to 271 of AMH epitope). AMH quality control substance was diluted with diluent to certain dilution values (20ng/ml, 10ng/ml, 5ng/ml, 2.5ng/ml, 1.25ng/ml, 0.5ng/ml, 0.1ng/ml, 0.05ng/ml, 0.01ng/ml). Sample serum, positive control, blank control in volume of 40ul were respectively added into corresponding wells, and incubated at 37°C for 40 minutes, the plate was washed for 5 times and added with F10G7-2-HRP (1/500 dilution) and incubated for 40 minutes, the plate was washed for 5 times, the substrate was added and incubated for 15 minutes. The values under wavelength of 450-620 were read by microplate reader, and the data analysis was shown in Table 7 below.

| Background value | C8H3-2 | C17B10 | G8B11 | 7D11-1 | C9F2-2 |
|---|---|---|---|---|---|
| 2.75 | 2.80 | 2.19 | 1.30 | 1.50 | 1.14 |
| 3.39 | 4.25 | 2.72 | 1.50 | 1.90 | 1.53 |
| 6.92 | 7.98 | 6.31 | 3.11 | 4.51 | 3.05 |
| 9.4 | 10.26 | 8.19 | 3.91 | 4.65 | 3.62 |
| 0.935 | 0.91 | 0.18 | 0.24 | 0.47 | 0.23 |
| 1.72 | 3.00 | 2.22 | 0.92 | 1.60 | 0.90 |
| 2.85 | 5.22 | 4.49 | 1.30 | 3.11 | 1.86 |
| 1.56 | 1.77 | 0.90 | 0.40 | 0.81 | 0.45 |
| 0.751 | 0.73 | -0.08 | 0.12 | 0.23 | 0.19 |
| 3.24 | 3.80 | 2.20 | 0.96 | 1.53 | 1.18 |
| 0.473 | 0.42 | -0.04 | 0.05 | 0.23 | 0.15 |
| 2.28 | 2.10 | 1.45 | 0.62 | 1.04 | 0.78 |
| 6.66 | 7.23 | 5.27 | 3.05 | 3.05 | 2.87 |
| 0.351 | 0.37 | -0.02 | 0.15 | 0.01 | -0.01 |
| 0.717 | 0.57 | -0.27 | 0.14 | 0.15 | 0.13 |
| 3.75 | 4.26 | 2.71 | 1.56 | 2.92 | 1.73 |
| 0.359 | 0.28 | -0.21 | 0.07 | 0.22 | 0.08 |
| 1.89 | 2.37 | 0.79 | 0.91 | 1.21 | 1.00 |
| 1.05 | 1.05 | -0.23 | 0.30 | 0.48 | 0.29 |
| 6.97 | 7.29 | 4.02 | 2.41 | 3.10 | 2.81 |
| 8.14 | 6.11 | 4.10 | 3.38 | 3.72 | 2.98 |
| 4.16 | 3.24 | 1.36 | 1.21 | 2.04 | 1.55 |
| 0.892 | 0.48 | 0.02 | 0.19 | 0.40 | 0.25 |
| 0.321 | 0.20 | -0.29 | 0.01 | -0.06 | -0.03 |
| 2.52 | 2.48 | 1.07 | 0.92 | 1.09 | 0.79 |
| 8.16 | 6.77 | 4.44 | 2.72 | 3.18 | 2.57 |
| 6.72 | 6.64 | 3.60 | 2.54 | 2.92 | 2.32 |
| 4.9 | 4.49 | 1.80 | 1.32 | 1.81 | 1.36 |
| 2.1 | 1.47 | -0.31 | 0.37 | 0.55 | 0.40 |
| 1.95 | 3.12 | 0.87 | 1.05 | 1.98 | 1.12 |
| 6.57 | 5.29 | 1.92 | 1.44 | 2.56 | 1.86 |
| 1.49 | 1.25 | 0.24 | 0.38 | 0.87 | 0.59 |
| 6.2 | 6.52 | 4.39 | 1.95 | 2.39 | 2.44 |
| 1.36 | 1.01 | -0.04 | 0.28 | 0.39 | 0.46 |
| 2.86 | 2.22 | 1.06 | 1.06 | 0.64 | 1.05 |
| 9.84 | 9.97 | 7.23 | 4.54 | 2.64 | 3.83 |
| 11.17 | 8.81 | 6.00 | 3.91 | 2.83 | 3.69 |
| 2.28 | 2.86 | 0.89 | 0.11 | 0.85 | 1.03 |
| | | | | | |
| r2 | 0.9164 | 0.8131 | 0.9212 | 0.7362 | 0.9386 |

2. The correlations of 38 sera were verified by enzyme immuno-pairing, as showing above, the specific monoclonal antibody C9F2-2 pairing showed good correlation.

### Example 8

Evaluation of monoclonal antibody pairing luminescence:
1. The AMH detection kit in the following example was characterized in that it comprised the following components: M reagent, containing 0.5-1 mg/ml magnetic particles coated with first antibody, wherein the first antibody (C9F2-2) had a coating amount of 20 to 80 ug/ml magnetic particles. The above-mentioned magnetic particles were purchased from Thermo Fisher Scientific, and the preparation method of this reagent comprised: the first antibody and magnetic particles were mixed in 2-morpholine/ethanesulfonic acid buffer with pH=5.0~6.0, coating was performed at 25°C to 37°C for 1 to 3 hours, then 0.1% to 0.5% bovine serum albumin in phosphate buffer with pH=8.0~9.0 was added and termination reaction was performed for 1 to 3 hours, the coated magnetic particles were separated and then dispersed in a phosphate buffer with pH of 7.0 to 8.0, then casein and bovine serum albumin were added. R1 reagent was the currently added system. R2 reagent comprised acridinium ester coated with a secondary antibody (monoclonal antibody F10G7-2, Xiamen Wantaikairui Biotechnology Co., Ltd., catalog number M3382), 0.5-1% casein and 0.5-1% bovine serum albumin, in which the solvent was phosphate buffer with pH=7.0~8.0, the second antibody had a coating amount of 5 to 15 ug/ml acridinium ester, and the preparation method of this reagent comprised: the second antibody and acridinium ester were mixed in phosphate buffer with pH=8.0~9.0, coating was performed at 25°C to 37°C for 1 to 3 hours, then pH=8.0~9.0 Tris buffer containing 0.1% to 0.5% bovine serum albumin was added and termination reaction was performed for 1 to 3 hours. A mother liquor was obtained therefore, and the mother liquor was diluted to 1-500 times with a phosphate buffer of pH=7.0-8.0 for use. The pre-excitation solution was 1% (w/v) hydrogen peroxide solution. The excitation solution was 1mol/L sodium hydroxide solution.
2. The detection method of the above AMH assay kit comprised the following steps: 100ul of sample R1 reagent in a volume ratio of 1:1 was added to each reaction well, incubated for 15 to 20 minutes, after the incubation, 0.05-0.08% Tween 20 phosphate buffer was used for washing, then 20 to 25 ul of R2 reagent was added, incubated for 10 to 15 minutes, after incubation, 0.05-0.08% Tween 20 phosphate buffer was used for washing, then 100 to 200ul of pre-excitation solution was added and pre-excitation was performed. The pre-excitation solution was removed, and 100 to 200ul of the excitation solution was added, then excitation and detection were performed. Wherein, (100) samples were tested and the correlations were as follows:

| Sample No. | Background value, ng/ml | Measured value |
|---|---|---|
| 1 | 6.27 | 6.83 |
| 2 | 4.75 | 4.73 |
| 3 | 6.53 | 7.29 |
| 4 | 1.75 | 1.46 |
| 5 | 7.23 | 8.49 |
| 6 | 3.75 | 4.22 |
| 7 | 1.15 | 0.87 |
| 8 | 1.43 | 1.48 |
| 9 | 3.05 | 3.11 |
| 10 | 2.66 | 2.51 |
| 11 | 2.79 | 3.18 |
| 12 | 1.35 | 1.27 |
| 13 | 1.05 | 1.04 |
| 14 | 3.39 | 3.56 |
| 15 | 2.52 | 3.33 |
| 16 | 5.66 | 6.36 |
| 17 | 1.21 | 1.22 |
| 18 | 5.53 | 6.05 |
| 19 | 3.35 | 3.17 |
| 20 | 12.53 | 16.34 |
| 21 | 0.449 | 0.46 |
| 22 | 1.14 | 1.23 |
| 23 | 3.72 | 4.13 |
| 24 | 1.28 | 1.04 |
| 25 | 3.07 | 4.57 |
| 26 | 0.83 | 0.92 |
| 27 | 4.33 | 5.15 |
| 28 | 3.91 | 4.94 |
| 29 | 2.21 | 2.47 |
| 30 | 2.29 | 2.37 |
| 31 | 1.93 | 2.16 |
| 32 | 1.4 | 1.18 |
| 33 | 0.866 | 0.58 |
| 34 | 0.324 | 0.34 |
| 35 | 1.47 | 1.65 |
| 36 | 1.49 | 1.35 |
| 37 | 3.49 | 3.44 |
| 38 | 2.27 | 2.17 |
| 39 | 0.693 | 0.57 |
| 40 | 4.08 | 3.07 |
| 41 | 3.37 | 5.15 |
| 42 | 1.51 | 1.61 |
| 43 | 4.54 | 5.42 |
| 44 | 1.34 | 1.54 |
| 45 | 4.97 | 6.41 |
| 46 | 1.59 | 1.84 |
| 47 | 4.21 | 4.75 |
| 48 | 3.73 | 3.60 |
| 49 | 1.89 | 1.70 |
| 50 | 9.22 | 10.53 |
| 51 | 6.72 | 8.87 |
| 52 | 4 | 6.31 |
| 53 | 4.8 | 7.47 |
| 54 | 3.46 | 3.38 |
| 55 | 4.65 | 3.81 |
| 56 | 0.38 | 0.34 |
| 57 | 5.59 | 6.60 |
| 58 | 2.83 | 4.77 |
| 59 | 0.088 | 0.11 |
| 60 | 4.99 | 6.78 |
| 61 | 2.23 | 2.86 |
| 62 | 4.24 | 4.69 |
| 63 | 3.83 | 4.22 |
| 64 | 2.79 | 2.52 |
| 65 | 3.09 | 2.76 |
| 66 | 0.754 | 0.69 |
| 67 | 0.775 | 0.73 |
| 68 | 2.67 | 2.94 |
| 69 | 1.73 | 1.66 |
| 70 | 1.08 | 1.39 |
| 71 | 4.16 | 4.57 |
| 72 | 5.56 | 7.72 |
| 73 | 2.19 | 1.90 |
| 74 | 3.97 | 3.39 |
| 75 | 4.29 | 3.95 |
| 76 | 2.95 | 2.88 |
| 77 | 8.34 | 9.02 |
| 78 | 7.47 | 5.70 |
| 79 | 2.63 | 2.39 |
| 80 | 0.5 | 0.78 |
| 81 | 4.1 | 3.22 |
| 82 | 1.53 | 1.48 |
| 83 | 0.363 | 0.37 |
| 84 | 6.94 | 8.43 |
| 85 | 0.805 | 1.75 |
| 86 | 2.24 | 2.77 |
| 87 | 0.561 | 0.61 |
| 88 | 5.9 | 7.86 |
| 89 | 1.76 | 2.13 |
| 90 | 1.11 | 1.10 |
| 91 | 3.09 | 3.03 |
| 92 | 1.53 | 2.08 |
| 93 | 2.35 | 2.97 |
| 94 | 3.1 | 3.54 |
| 95 | 1.95 | 1.81 |
| 96 | 2.25 | 2.64 |
| 97 | 5.19 | 6.40 |
| 98 | 1.63 | 0.68 |
| 99 | 3.22 | 2.99 |
| 100 | 1.28 | 1.48 |

3. 100 Serum samples were detected by the luminescence kit, and the correlation R2 with the control reagent could reach 0.93.

## Claims

1. A monoclonal antibody that specifically binds to human anti-Mullerian hormone, wherein the antibody specifically binds to an epitope of anti-Mullerian hormone identical to that to which an antibody C9F2-2 produced by the hybridoma with access number of CCTCC NO: C2018183 binds.

2. A monoclonal antibody that specifically binds to human anti-Mullerian hormone, wherein the antibody comprises a light chain variable region complementarity determining region (CDR) and a heavy chain variable region CDR of an antibody C9F2-2 produced by the hybridoma with access number of CCTCC NO: C2018183.

3. A monoclonal antibody that specifically binds to human anti-Mullerian hormone, wherein the antibody comprises a light chain variable region and a heavy chain variable region, the light chain variable region comprises a CDR1 with an amino acid sequence of SEQ ID NO: 4, a CDR2 with an amino acid sequence of SEQ ID NO: 5 and a CDR3 with an amino acid sequence of SEQ ID NO: 6, and the heavy chain variable region comprises a CDR1 with an amino acid sequence of SEQ ID NO: 1, a CDR2 with an amino acid sequence of SEQ ID NO: 2 and a CDR3 with an amino acid sequence of SEQ ID NO: 3.

4. The monoclonal antibody according to claim 3, wherein the antibody comprises: (a) a light chain variable region having an amino acid sequence of SEQ ID NO: 8 and a heavy chain variable region having an amino acid sequence of SEQ ID NO: 7.

5. A monoclonal antibody that specifically binds to human anti-Mullerian hormone, wherein the antibody comprises an antigen-binding region, and the antigen-binding region is derived from light chain and heavy chain variable regions of an antibody C9F2-2 produced by the hybridoma with access number of CCTCC NO: C2018183.

6. The monoclonal antibody according to any one of claims 1 to 5, which is an antigen-binding fragment, a single chain antibody, a chimeric antibody, a monovalent antibody, a multispecific antibody, a human antibody, or a Fab fragment.

7. A monoclonal antibody that specifically binds to human anti-Mullerian hormone, wherein the antibody is obtained from the hybridoma with access number of CCTCC NO: C2018183.

8. A monoclonal antibody against human anti-Mullerian hormone, which specifically binds to an epitope contained in a sequence spanning amino acid residues 38 to 45 of anti-Mullerian hormone.

9. The hybridoma cell line with access number of CCTCC NO: C2018183.

10. A polynucleotide encoding a heavy chain variable region and/or a light chain variable region of the antibody against anti-Mullerian hormone according to any one of claims 1 to 8.

11. A vector comprising the polynucleotide according to claim 10.

12. A host cell comprising the polynucleotide according to claim 11 or the vector according to claim 11.

13. A method for detecting the presence or a level of human anti-Mullerian hormone in a sample, which comprises a step of using the antibody or antigen-binding fragment thereof according to any one of claims 1 to 8.

14. A use of the antibody or antigen-binding fragment thereof according to any one of claims 1 to 8 in manufacture of a kit for detecting the presence or a level of human anti-Mullerian hormone in a sample.

15. A method for detecting AMH or a fragment thereof in a biological sample, the method comprising:
allowing the sample to contact with at least two antibodies or antigen-binding fragments thereof against different epitopes of anti-Mullerian hormone, and qualitatively or quantitatively detecting the binding of the at least two antibodies to anti-Mullerian hormone or the fragments, wherein the binding indicates the presence or a concentration of anti-Mullerian hormone or the fragment in the sample;
wherein at least one antibody or antigen-binding fragment thereof is directed against an epitope contained in a sequence of amino acid residues 38 to 45 of anti-Mullerian hormone.

16. The method according to claim 15, wherein one antibody or antigen-binding fragment thereof is directed against an epitope contained in a sequence of amino acid residues 38 to 45 of anti-Mullerian hormone, and another antibody or antigen-binding fragment thereof is directed against an epitope contained in a sequence of amino acid residues 358 to 369 of anti-Mullerian hormone, or an epitope contained in a sequence of amino acid residues 491 to 502 of anti-Mullerian hormone, or an epitope contained in a sequence of amino acid residues 260 to 271 of anti-Mullerian hormone, or an epitope contained in a sequence of amino acid residues 330 to 343 of anti-Mullerian hormone.

17. The method according to claim 15, wherein at least one of the at least two antibodies or antigen-binding fragments thereof is immobilized on a solid surface, preferably, at least one of another or other antibodies is labeled, and the labeling is preferably carried out by covalently attaching a chemiluminescent or fluorescent dye.

18. The method according to claim 15 or 16, wherein an antibody or antigen-binding fragment thereof against an epitope contained in a sequence spanning amino acid residues 38 to 45 of anti-Mullerian hormone is immobilized on a solid surface, and another antibody or antigen-binding fragment thereof is directed against an epitope contained in a sequence of amino acid residues 260 to 271 of anti-Mullerian hormone.

19. A kit for detecting the presence or a level of anti-Mullerian hormone in a sample, which comprises the antibody or antigen-binding fragment thereof according to any one of claims 1 to 8, which is used as a first monoclonal antibody, i.e., a coating antibody, for double-antibody sandwich enzyme-linked immunoassay; and a second monoclonal antibody, i.e., an enzyme-labeled antibody, for double-antibody sandwich enzyme-linked immunoassay, wherein the coating antibody and the enzyme-labeled antibody are respectively directed against different antigenic determinants of anti-Mullerian hormone;
preferably, the enzyme-labeled antibody is directed against an epitope contained in a sequence of amino acid residues 260 to 271 of anti-Mullerian hormone.

20. The kit according to claim 19, which further comprises one or more of the followings: an enzyme-linked plate, a coating buffer, a blocking solution, a chromogenic substrate, a stop solution, an anti-Mullerian hormone standard.
